(19) Europäisches Patentamt
European Patent Office
Office européen des brevets

(11) Numéro de publication: 0 161 132
B1

(12) FASCICULE DE BREVET EUROPÉEN

(45) Date de publication du fascicule du brevet: 20.05.87

(51) Int. Cl.⁴: C 07 C 39/19, C 07 C 39/225, C 07 C 39/373, C 07 C 43/23, C 07 C 69/84, C 07 C 37/14, C 07 C 41/30, C 07 C 67/347

(21) Numéro de dépôt: 85400538.6

(22) Date de dépôt: 21.03.85

# (54) Procédé de C-alcoylation selective des phenols.

(30) Priorité: 22.03.84 FR 8404443

(43) Date de publication de la demande: 13.11.85 Bulletin 85/46

(45) Mention de la délivrance du brevet: 20.05.87 Bulletin 87/21

(84) Etats contractants désignés: AT BE CH DE FR GB IT LI LU NL SE

(56) Documents cité:
US-A-3 518 318

CHEMICAL ABSTRACTS, vol. 88, no. 17, 24 avril 1978, page 509, no. 120776w, Columbus, Ohio, US;
CHEMICAL ABSTRACTS, vol. 93, no. 17, 27 octobre 1980, page 664, no. 168127r, Columbus, Ohio, US;

(73) Titulaire: RHONE- POULENC SANTE, Les Miroirs 18 Avenue d'Alsace, F-92400 Courbevoie Cedex (FR)

(72) Inventeur: Mignani, Gérard, 2 avenue des Frères Lumière, F-69008 Lyon (FR)
Inventeur: Morel, Didier, 19 avenue Jean Mermoz, F-69008 Lyon (FR)

(74) Mandataire: Pilard, Jacques, RHONE- POULENC RECHERCHES Service Brevets Pharma 25, Quai Paul Doumer, F-92408 Courbevoie Cedex (FR)

EP 0 161 132 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne un procédé de préparation de phénols substitués de formule générale:

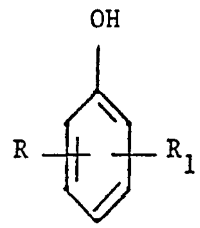 (I)

dans laquelle
$R_1$ représente un radical

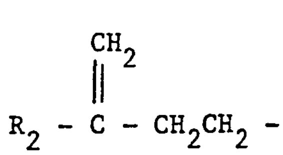 ou 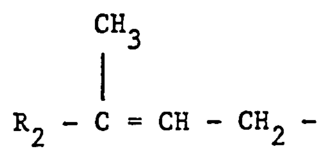

dans lequel $R_2$ représente un radical choisi parmi les radicaux acycliques ayant de 1 à 20 atomes de carbone éventuellement substitués par un ou plusieurs radicaux hydroxy, alcoyloxycarbonyles, cyano ou formyle, et les radicaux phényle ou naphtyle éventuellement substitués par un ou plusieurs radicaux alcoyles contenant 1 à 20 atomes de carbone,

et R représente un atome d'hydrogène ou 1 à 4 substituants, identiques ou différents, choisis parmi les atomes d'halogène, les radicaux hydroxy éventuellement sous forme d'éthers ou d'esters, alcoyles, nitro, formyle éventuellement sous forme d'acétal, acétyle, benzoyle, amino, alcoylamino, dialcoylamino ou alcoyloxycarbonyle, étant entendu que deux des symboles R peuvent former avec le noyau phényle un cycle aromatique condensé tel qu'un cycle naphtyle et étant entendu que le substituant $R_1$ est en position ortho ou para de la fonction phénol, par action d'un butadiène de formule générale:

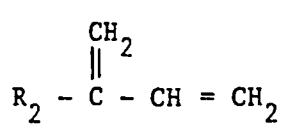 (II)

sur un phénol de formule générale

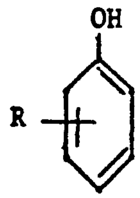

(III)

dans laquelle R est défini comme précédemment, étant entendu qu'au moins une position en ortho ou para du radical hydroxy n'est pas substituée.

Comme exemples de radicaux acycliques peuvent être cités les radicaux $CnH_{2n+1}$,$CnH_{2n-1}$, $CnH_{2n-3}$ et $CnH_{n-1}$ où n est compris entre 1 et 20 comme par exemple $-CH_3$ (isoprène); $-C_2H_5$; $-C_4H_9$; $-C_8H_{17}$; $-C_{20}H_{41}$; $-C_4H_7$; $C_6H_{11}$ (myrcène); $-C_8H_{15}$; $-C_{10}H_{19}$; $-C_8H_{13}$; $-C_{11}H_{19}$ (β-farnésène); $-C_2H$, $-C_4H_3$; $-C_8H_7$, le nom commun des composés de formule (II) les plus importants étant indique entre parenthèses.

Comme exemples de radicaux phényle et naphtyle éventuellement substitués peuvent être cités les radicaux tolyle, xylyle, méthyl-8 naphtyle-1 et diméthyl-5,8 naphtyle-2.

L'alcoylation des phénols par les butadiènes conjugués de formule générale (II) est une réaction connue. Cependant les procédés décrits dans la littérature conduisent à l'obtention de mélanges dont les constituants peuvent être difficilement séparés.

Ainsi, d'après A.R. Bader et W.C. Bean, J. Amer. Chem. Soc., 80, 3073 (1958), la réaction du phénol avec l'isoprène catalysée par l'acide phosphorique à 71 % à 20° C conduit à un mélange de 6 produits.

D'après K.C. Dewhirst et F.F Rust, J. Org. Chem., 28, 798 (1963), le traitement du phenol par l'isoprène en présence de phénate d'aluminium à basse température conduit à un mélange de 4 produits dont la composition varie considérablement avec les conditions de la réaction. Cependant il n'est pas possible d'orienter la réaction vers l'obtention d'un produit unique.

D'après L. Bolzoni et coll., Angew. Chem. Int. Ed. 17 (9) 684 (1978), l'isoprène ou le myrcène réagissent avec le phénol en présence de phénate de potassium et de chlorure d'aluminium pour donner des chromanes. L'utilisation d'acide phosphorique comme catalyseur peut conduire également, selon V.K. Ahluwalia et K.K. Arora, Tetrahedron 17, 1437 (1981) à l'obtention de chromanes.

Dans le brevet américain US 4 168 271 est décrite la condensation du phytatriène avec la triméthylhydroquinone en présence de chlorure cuivreux et de chlorure d'aluminium dans un mélange benzène-acétate d'éthyle qui conduit au tocophérol avec un rendement de 47,2 %.

Il est connu également, d'après E.J. Smutny, Ann. New-York Acad. Sci., 125 (1973), d'effectuer la condensation du phénol avec le butadiène en présence de complexes du palladium. Il se forme, avec des rendements modérés (30 à 50 %) et un taux de conversion faible (5 à 10 %) un mélange d'ortho et para buténylphénols et de buténylphényléther.

Enfin, d'après la demande de brevet japonais publiée sous le numéro 55 015 411, des dialcoxy-3,4 phénols peuvent être condensés avec des diènes conjugués, en particulier l'isoprène en présence d'un catalyseur à base de rhodium et d'une phosphine dans un solvant organique pour donner des dialcoxy-3,4 phénols C-alcoylés en ortho. Un inconvénient particulier de ce procédé d'alcoylation en phase liquide, dans lequel les systèmes catalytiques utilisés sont homogènes et en solution, réside dans le fait qu'il est nécessaire d'effectuer un traitement supplémentaire difficile en vue de séparer les produits de la réaction de la solution catalytique et de séparer le catalyseur pour le recycler. Par exemple, le catalyseur peut être recyclé dans le résidu lourd obtenu après distillation des produits de la réaction. Un tel traitement de la solution catalytique ne peut être appliqué que lorsque les produits de la réaction sont volatils, les solutions catalytiques se décomposant facilement durant le traitement en précipitant le métal sous sa forme métallique. Le système catalytique perd donc de son efficacité ce qui diminue l'intérêt industriel du procédé.

Il est donc souhaitable de pouvoir disposer d'un procédé d'alcoylation en phase liquide en présence d'un catalyseur à base de rhodium qui permette de surmonter les inconvénients des procédés antérieurs, en particulier au niveau de la récupération du catalyseur, tout en conservant les avantages des catalyseurs homogènes en phase liquide qui permettent d'obtenir des rendements et des sélectivités élevés.

Il a maintenant été trouvé, et c'est ce qui fait l'objet de la présente invention, que la C-alcoylation d'un phénol de formule générale (III) par un butadiène de formule générale (II) peut être effectuée sélectivement en opérant dans l'eau en présence d'un catalyseur à base de rhodium et d'une phosphine soluble dans l'eau, et éventuellement en présence d'une base, sans obtenir de produit de O-alcoylation.

D'un intérêt tout particulier sont les dérivés du butadiène de formule générale (II) choisis parmi l'isoprène et le myrcène.

Le composé du rhodium utilisé doit être soluble dans l'eau ou capable de passer en solution dans l'eau dans les conditions de la réaction, par réaction de coordination avec les phosphines hydrosolubles. Le reste lié au

métal n'est pas critique dès lors qu'il satisfait a ces conditions.

Le dérivé du rhodium est choisi généralement dans le groupe comprenant les sels organiques ou inorganiques et les complexes du rhodium tels que $RhCl_3$, $RhBr_3$, $Rh_2O$, $Rh_2O_3$, $Rh(NO_3)_3$, $Rh_2(CH_3COO)_4$, $Rh(CH_3COCHCOCH_3)_3$, $[RhCl(cyclooctadiène\text{-}1,5)]_2$, $[RhCl(CO)_2]_2$ ou $RhCl_3(C_2H_5NH_2)_3$. D'un intérêt tout particulier sont $RhCl_3$ et $[RhCl(cyclooctadiène\text{-}1,5)]_2$.

Les phosphines solubles dans l'eau qui conviennent particulièrement bien sont celles décrites dans le brevet français FR 7 622 824 publié sous le numéro 2 366 237.

Plus particulièrement, est utilisée au moins une phosphine de formule générale

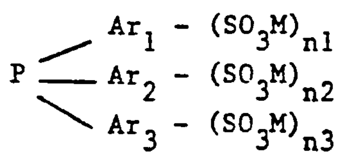

(IV)

dans laquelle:

- $A_{r1}$, $Ar_2$ et $Ar_3$, identiques ou différents, représentent chacun un radical choisi dans le groupe des radicaux phénylène et naphtylène éventuellement substitués,

- M est un reste cationique minéral ou organique choisi de telle manière que la phosphine de formule générale (IV) soit soluble dans l'eau,

- $n_1$, $n_2$ et $n_3$, identiques ou différents, sont des nombres entiers supérieurs ou égaux à 0 et inférieurs ou égaux à 3, l'un au moins étant supérieur ou égal à 1.

Les radicaux phénylène et naphtalène peuvent être substitués, par exemple, par un ou plusieurs atomes ou radicaux choisis parmi les atomes d'halogène et les radicaux hydroxy, cyano, nitro, dialcoylamino ou alcoyloxycarbonyles.

De préférence, on utilise au moins une phosphine de formule générale (IV) dans laquelle $Ar_1$, $Ar_2$ et $Ar_3$, identiques ou différents, représentent chacun un radical phénylène éventuellement substitué et dans laquelle au moins un des groupements $SO_3M$ est en position méta sur le noyau benzénique.

De préférence M est choisi parmi les cations sodium, potassium, calcium, baryum, ammonium ou ammonium quaternaires tels que tétrapropylammonium ou tétrabutylammonium.

De préférence, $n_1$, $n_2$ et $n_3$ sont égaux à 0 ou 1, $n_1 + n_2 + n_3$ étant compris entre 1 et 3.

Comme phosphines de formule générale (IV) pouvant être utilisées dans le procédé selon l'invention peuvent être cités les sels alcalins ou alcalino-terreux, les sels d'ammonium et les sels d'ammonium quaternaires des (p-sulfophényl) diphénylphosphine; (m-sulfo p-méthylphényl) di(p-méthylphényl) phosphine; (m-sulfo p-méthoxyphényl) di(p-méthoxyphényl) phosphine; (m-sulfo p-chlorophényl) di(p-chlorophényl) phosphine; di(p-sulfophényl) phénylphosphine; di(m-sulfo p-méthylphényl) (p-méthylphényl) phosphine; di(m-sulfo p-méthoxyphényl) (p-méthoxyphényl) phosphine; di(m-sulfo p-chlorophényl) (p-chlorophényl) phosphine; tri(p-sulfophényl) phosphine; tri(m-sulfo p-méthylphényl) phosphine; tri(m-sulfo p-méthoxyphényl) phosphine; tri(m-sulfo p-chlorophényl) phosphine; tri(o-sulfo p-méthylphényl) phosphine; (o-sulfo p-méthylphényl) (m-sulfo p-méthylphényl) (m,m'-disulfo p-méthylphényl) phosphine; (m-sulfophényl) (m-sulfo p-chlorophényl) (m,m'-disulfo p-chlorophényl) phosphine.

On utilise une quantité de rhodium ou de composé du rhodium telle que le nombre d'atomes-gramme de rhodium élémentaire par litre de solution reactionnelle soit compris entre $10^{-4}$ et 1, de préférence entre $10^{-3}$ et 0,5.

La quantité de phosphine est choisie de telle sorte que le nombre d'atomes-gramme de phosphore trivalent rapporté à un atome-gramme de rhodium soit compris entre 0,1 et 200, de préférence entre 3 et 100.

La quantité d'eau minimum nécessaire est celle suffisante pour dissoudre le catalyseur en totalité et au moins une partie des produits de formule générale (II) et (III), la réaction ayant lieu en phase aqueuse et les produits de la réaction étant en phase organique non miscible à l'eau.

Selon un mode de mise en oeuvre particulier mais non obligatoire de l'invention, afin d'augmenter la cinétique de la réaction et de pouvoir recycler plus facilement le catalyseur, il est possible d'opérer en présence d'un co-solvant.

Plus particulièrement, une partie de l'eau nécessaire à la mise en oeuvre de la réaction est remplacée par une quantité équivalente d'un alcool aliphatique contenant 1 à 4 atomes de carbone tel que le méthanol, l'éthanol, l'isopropanol ou le butanol. La quantité d'eau qui peut être remplacée est au maximum égale à la moitié de la quantité d'eau nécessaire à la mise en oeuvre de la réaction sans co-solvant.

Pour améliorer la réactivité, on peut ajouter une base au mélange réactionnel. Comme bases convenant particulièrement bien peuvent être citées les hydroxydes, carbonates et bicarbonates de métaux alcalins ou

alcalino-terreux et les amines tertiaires aliphatiques ou aromatiques. On utilise généralement de 0,005 à 5 moles de base par litre de solution aqueuse.

Généralement, on utilise un léger excès molaire de butadiène de formule (II) par rapport au phénol de formule générale (III).

Généralement la réaction est mise en oeuvre à une température inférieure à 200°C et de préférence entre 50 et 125°C.

Pour la mise en oeuvre du procédé selon l'invention, il est possible de charger dans un réacteur convenable préalablement purgé au moyen d'un gaz inerte (azote, argon) soit la solution catalytique préalablement préparée soit les divers composants (phosphine, eau, composé du rhodium et éventuellement la base). Le réacteur est chauffé à la température de réaction avant ou après l'introduction du phénol qui est lui-même introduit avant ou après ou simultanément avec le butadiène de formule générale (II).

Lorsque la réaction est terminée, le réacteur est refroidi à une température voisine de 20°C. Le contenu du réacteur est soutiré et le produit de formule générale (I) est isolé de la phase organique par décantation et éventuellement par extraction par un solvant convenable tel que l'éther diéthylique, le pentane, l'hexane.

Le produit de formule générale (1) peut être séparé, par exemple, par distillation.

Les produits de formule générale (I) peuvent trouver application dans de nombreux domaines. En particulier, les produits de formule générale (I) convenablement substitués peuvent être utilisés comme intermédiaires dans la synthèse de la vitamine E ou d'antioxydants [.W. Burton et K.U. Ingold, J. Amer. Chem. Soc., 103, 6472 (1981)], de parfums (brevet américain US 4 400 545) ou d'insecticides [P. Barua et coll., Tetrahedron Letters, 24 (51) 5801 (1983)].

Les exemples suivants, donnés à titre non limitatif, montrent comment l'invention peut être mise en pratique.

**EXEMPLE 1**

Dans un autoclave en acier inoxydable de 125 $cm^3$, on introduit 0,0639 g de $[RhCl(cyclooctadiène\text{-}1,5)]_2$ soit 0,26 milliatome-gramme de rhodium, 0,9246 g de tri(m-sulfophényl) phosphine sous forme de sel de sodium (désigné ci-après par TPPTS Na) soit 1,37 milli-atome-gramme de $P^{3+}$ et 0,2484 g de carbonate de sodium (2,3 m.moles). On purge trois fois à l'argon puis on ajoute successivement 16 g d'eau distillée, 18,37 g de phénol (0,195 mole) et 13,94 g d'isoprène (0,205 mole). On chauffe à 100°C puis agite pendant 6 heures.

Après refroidissement et décantation, la phase organique est lavée à l'eau. On recueille ainsi, après séchage, sur sulfate de sodium, filtration et concentration à 30°C sous pression réduite (15 mm de mercure; 2 kPa), une phase organique pesant 6,31 g contenant:

30 % de (méthyl-3 butène-3 yl)-2 phénol
65 % de (méthyl-3 butène-3 yl)-4 phénol
et 5 % de (méthyl-3 butène-3 yl)-3 phénol.

Le taux de transformation de l'isoprène est de 20 %.

**EXEMPLE 2**

Dans un autoclave en acier inoxydable de 125 $cm^3$, on introduit 0,0302 g de $[RhCl(cyclooctadiène\text{-}1,5)]_2$ soit 0,122 milli-atome-gramme de rhodium, 1 g de TPPTS Na soit 1,48 milli-atome-gramme de phosphore et 0,025 g de carbonate de sodium (0,23 m.mole). On purge trois fois à l'argon puis on ajoute successivement 15 g d'eau distillée, 12,4 g de gaïacol (méthoxy-2 phénol) soit 0,1 mole et 7,02 g d'isoprène (0,103 mole). Om chauffe à 100°C puis on agite pendant 6 heures.

Après refroidissement et décantation la phase organique est lavée à l'eau. Après séchage sur sulfate de sodium, filtration et concentration, on obtient une phase organique pesant 2,67 g contenant:

72 % de (méthyl-3 butène-3 yl)-4 méthoxy-2 phénol
10 % de (méthyl-3 butène-2 yl)-4 méthoxy-2 phénol
14 % de (méthyl-3 butène-3 yl)-6 méthoxy-2 phénol.
4 % de (méthyl-3 butène-2 yl)-6 méthoxy-2 phénol.

Le taux de transformation de l'isoprène est de 13,5 %.

**EXEMPLE 3**

Dans un autoclave en acier inoxydable de 125 $cm^3$, on introduit 0,1 g de $[RhCl(cyclooctadiène\text{-}1,5)]_2$ soit 0,406 milli-atome-gramme de rhodium, 1,6 g de TPPTS Na soit 2,03 milli-atome-gramme de phosphore, 0,252 g de carbonate de sodium (2,37 m.moles) et 8 g de pyrocatéchine (72,6 m.moles). On purge trois fois à l'argon puis on ajoute successivement 20 g d'eau distillée et 10,2 g d'isoprène (0,15 mole). On chauffe à 100°C puis on agite pendant 6 heures.

Après refroidissement, décantation et concentration, on recueille 13,94 g de phase organique.

Le taux de transformation de l'isoprène est de 58 % et celui de la pyrocatéchine de 100 %.

L'analyse chromatographique de la phase organique montre que le produit obtenu contient 68 % de produit de monoaddition [(méthyl-3 butène-2 yl)-3 hydroxy-2 phénol (40 %) et (méthyl-3 buténe-3 yl)-3 hydroxy-2 phénol (60 %)] et 32 % de produit de diaddition.

La structure des produits obtenus est confirmée par le spectre infra-rouge, le spectre de résonance magnétique nucléaire du proton et le spectre de masse.

## EXEMPLE 4

Dans un autoclave en acier inoxydable de 125 $cm^3$, on introduit 0,10 g de $[RhCl(cyclooctadiène-1,5)]_2$ soit 0,406 milli-atome-gramme de rhodium, 1,6 g de TPPTS Na soit 2,03 milli-atome-gramme de phosphore, 0,252 g de carbonate de sodium (2,37 m.moles) et 8 g d'hydroquinone (72,6 m.moles). On purge trois fois à l'argon puis on ajoute successivement 20 g d'eau distillée et 5,44 g d'isoprène (80 m.moles).On chauffe à 100° C puis on agite pendant 5 heures.

Après refroidissement, décantation et concentration, on obtient 11,83 g de phase organique.

Le taux de transformation de l'isoprène est de 70,4 % et celui de l'hydroquinone de 57 %.

L'analyse chromatographique de la phase organique montre que le produit obtenu contient 82 % de produits de monoaddition [(méthyl-3 butène-2 yl)-2 hydroquinone (20 %) et (méthyl-3 butène-3 yl)-2 hydroquinone (80 %)] et 18 % de produit de diaddition.

La structure des produits obtenus est confirmée par le spectre infra-rouge, le spectre de résonance magnétique nucléaire du proton et le spectre de masse.

## EXEMPLE 5

Dans un autoclave en acier inoxydable de 125 $cm^3$, on introduit 0,10 g de $[RhCl(cyclooctadiène-1,5)]_2$ soit 0,406 milli-atome-gramme de rhodium, 1,6 g de TPPTS Na soit 2,03 milli-atome-gramme de phosphore, 0,252 g de carbonate de sodium (2,37 m.moles) et 5,616 g de triméthylhydroquinone (TMHQ) (36,9 m.moles). On purge trois fois à l'argon puis on ajoute 20 g d'eau distillée et 3,06 g d'isoprène (45 m.moles). On chauffe à 100° C puis on agite pendant 6 heures.

Après refroidissement, décantation et concentration, on recueille 5,5 g de phase organique qui cristallise sous forme d'un solide blanchâtre.

Le taux de transformation de l'isoprène est de 14,7 % et celui de la TMHQ de 18 %.

L'analyse chromatographique du solide obtenu montre qu'il contient:

10 % de (méthyl-3 butène-2 yl)-2 triméthyl-3,5,6 hydroquinone,

80 % de (méthyl-3 butène-3 yl)-2 trimethyl-3,5,6 hydroquinone

et 10 % de produit O-alcoylé.

La structure des produits obtenus est confirmée par le spectre infra-rouge, le spectre de résonance magnétique nucléaire du proton et le spectre de masse.

## EXEMPLE 6

Dans un autoclave en acier inoxydable de 125 $cm^3$, on introduit 0,10 g de $[RhCl(cyclooctadiène-1,5)]_2$ (0,406 milli-atome-gramme de rhodium, 1,6 g de TPPTS Na (2,56 milli-atome-gramme de $P^{3+}$), 0,251 g de carbonate de sodium (2,36 m.moles) et 8,02 g de résorcinol (72,8 m.moles) (métahydroxyphénol). On purge trois fois à l'argon et on ajoute 20 g d'eau distillée et 5,44 g d'isoprène (80 m.moles). On agite à 100° C pendant 6 heures.

Après refroidissement, décantation et concentration à 30° C sous pression réduite (15 mm de mercure; 2 kPa), on recueille 14,37 g de liquide jaune huileux.

Le taux de transformation de l'isoprène est de 100 % et celui du résorcinol de 90,7 %.

L'analyse chromatographique de l'huile montre la présence de 90 % de produits de monoaddition [hydroxy-3 (méthyl-3 butène-2 yl)phénol (45 %) et hydroxy-3 (méthyl-3 butène-3 yl)phénol, (55 %)] et 10 % de produits de diaddition.

La structure des produits obtenus est confirmée par le spectre de résonance magnétique nucléaire du proton et le spectre de masse.

**EXEMPLE 7**

Dans un autoclave en acier inoxydable de 125 $cm^3$, on introduit 0,10 g de $[RhCl(cyclooctadiène\text{-}1,5)]_2$ (0,406 milli-atome-gramme de rhodium, 1,6 g de TPPTS Na (2,56 milli-atome-gramme de $P^{3+}$), 0,251 g de carbonate de sodium (2,36 m.moles) et 10,54 g de naphtol-2 (72,8 m.moles). On purge trois fois à l'argon et on ajoute 20 g d'eau distillée et 5,44 g d'isoprène (80 m.moles). On agite à 100° C pendant 16 heures.

Après refroidissement, décantation et concentration à 30°C sous pression réduite (15 mm de mercure; 2 kPa), on recueille 15,45 g de solide de couleur beige.

Le taux de transformation de l'isoprène est de 88 % et celui du naphtol-2 de 94 %.

L'analyse chromatographique du solide montre la présence de 97 % de produits de monoaddition [(méthyl-3 butène-3 yl)-1 naphtol-2 (30 %) et (méthyl-3 butène-2 yl)-1 naphtol (70 %)] et 3 % de produits de diaddition.

La structure des produits obtenus est confirmée par le spectre de résonance magnétique nucléaire du proton et par le spectre de masse.

**EXEMPLE 8**

Dans un autoclave en acier inoxydable de 125 $cm^3$, on introduit 0,10 g de $[RhCl(cyclooctadiène\text{-}1,5)]_2$ (0,406 milli-atome-gramme de rhodium), 1,23 g de TPPTS Na (2 milli-atome-gramme de $P^{3+}$), 0,251 g de carbonate de sodium (2,36 m.moles) et 7,96 g de paracrésol (73,6 m.moles). On purge trois fois à l'argon et on ajoute 20 g d'eau distillée et 5,44 g d'isoprène (80 m.moles). On agite à 100° C pendant 16 heures.

Après refroidissement, décantation et concentration à 30°C sous pression réduite (15 mm de mercure; 2 kPa), on recueille 10,07 g de liquide orangé huileux.

Le taux de transformation de l'isoprène est de 56 % et celui du paracrésol de 60 %.

L'analyse chromatographique du solide montre la présence de 98 % de produits de monoaddition [méthyl-4 (méthyl-3 butène-3 yl)-2 phénol (80 %) et méthyl-4 (méthyl-3 butène-2 yl)-2 phénol (20 %)] et 2 % de produits de diaddition.

La structure des produits obtenus est confirmée par le spectre de résonance magnétique nucléaire du proton et par le spectre de masse.

**EXEMPLE 9**

Dans un autoclave en acier inoxydable de 125 $cm^3$, on introduit 0,1 g de $[RhCl(cyclooctadiène\text{-}1,5)]_2$ (0,406 milli-atome-gramme de rhodium), 1,23 g de TPPTS Na (2 milli-atome-gramme de $P^{3+}$), 0,251 g de carbonate de sodium (2,36 m.moles) et 9,77 g de chloro-4 phénol (76 m.moles). On purge trois fois à l'argon et on ajoute 20 g d'eau distillée et 5,44 g d'isoprène (80 m.moles). On agite à 100° C pendant 16 heures.

Après refroidissement, décantation et concentration à 30°C sous pression réduite (15 mm de mercure; 2 kPa), on recueille 11,01 g de liquide brun-rouge huileux.

Le taux de transformation de l'isoprène est de 26,3 % et celui du chloro-4 phénol de 21,3 %.

L'analyse chromatographique de l'huile montre la présence de 100 % de produits de monoaddition [chloro-4 (méthyl-3 butène-2 yl)-2 phénol (17 %) et chloro-4 (méthyl-3 butène-3 yl)-2 phénol (83 %)].

La structure des produits obtenus est confirmée par le spectre de résonance magnétique nucléaire du proton.

**EXEMPLE 10**

Dans un autoclave en acier inoxydable de 125 $cm^3$, on introduit 0,1 g de $[RhCl(cyclooctadiène\text{-}1,5)]_2$ (0,406 milli-atome-gramme de rhodium), 1,23 g de TPPTS Na (2 milli-atome-gramme de $P^{3+}$), 0,251 g de carbonate de sodium (2,36 m.moles) et 11,05 g de parahydroxybenzoate de méthyle (72,6 m.moles). On purge trois fois à l'argon et on ajoute 20 g d'eau distillée et 5,44 g d'isoprène (80 m.moles), On agite à 100° C pendant 16 heures.

Après refroidissement, décantation et concentration à 30°C sous pression réduite (15 mm de mercure; 2 kPa), on recueille 10,06 g de solide.

Le taux de transformation de l'isoprène est de 8,5 % et celui du parahydroxybenzoate de méthyle de 9,4 %.

L'analyse chromatographique du solide montre la présence de 100 % de produits de monoaddition [hydroxy-4 (méthyl-3 butène-2 yl)-3 benzoate de méthyle (33 %) et hydroxy-4 (méthyl-3 butène-3 yl)-3 benzoate de méthyle (67 %)].

La structure des produits obtenus est confirmés par le spectre de résonance magnétique nucléaire du proton.

**EXEMPLE 11**

Dans un autoclave en acier inoxydable de 125 $cm^3$, on introduit 0,1 g de $[RhCl(cyclooctadiène-1,5)]_2$ (0,406 milli-atome-gramme de rhodium), 1,23 g de TPPTS Na (2 milli-atome-gramme de $P^{3+}$), 0,251 g de carbonate de sodium (2,36 m.moles) et 7,92 g d'amino-4 phénol (72,55 m.moles). On purge trois fois à l'argon et on ajoute 20 g d'eau distillée et 5,44 g d'isoprène (80 m.moles). On agite à 100°C pendant 16 heures.

Après refroidissement, décantation et concentration à 30°C sous pression réduite (15 mm de mercure; 2 kPa), on recueille 11,86 g de solide de couleur rousse.

Le taux de transformation de l'isoprène est de 83,6 % et celui de l'amino-4 phénol de 93 %.

L'analyse chromatographique montre la présence de 100 % de produits de monoaddition [amino-4 (méthyl-3 butène-2 yl)-2 phénol (40 %) et amino-4 (méthyl-3 butène-3 yl)-2 phénol (60 %)].

La structure des produits obtenus est confirmée par le spectre de résonance magnétique nucléaire du proton.

**EXEMPLE 12**

Dans un autoclave en acier inoxydable de 125 $cm^3$, on introduit 0,1 g de $[RhCl(cyclooctadiène-1,5)]_2$ (0,406 milli-atome-gramme de rhodium), 1,23 g de TPPTS Na (2 milli-atome-gramme de $P^{3+}$) et 0,251 g de carbonate de sodium (2,36 m.moles). On purge trois fois à l'argon et on ajoute 20 g d'eau distillée, 9,33 g de chloro-2 phénol (72,6 m.moles) et 5,44 g d'isoprène (80 m.moles). On agite à 100°C pendant 16 heures.

Après refroidissement, décantation et concentration à 30°C sous pression réduite (15 mm de mercure; 2 kPa), on recueille 12,58 g de liquide jaune fluide.

Le taux de transformation de l'isoprène est de 76,6 % et celui du chloro-2 phénol de 83,8 %.

L'analyse chromatographique montre la présence de 100 % de produits de monoaddition [chloro-2 (méthyl-3 butène-2 yl)-4 phénol (20 %), chloro-2 (méthyl-3 butène-3 yl)-4 phénol (65 %), chloro-2 (méthyl-3 butène-2 yl)-6 phénol (5 %) et chloro-2 (méthyl-3 butène-3 yl)-6 phénol (10 %)].

La structure des produits obtenus est confirmée par le spectre de résonance magnétique nucléaire du proton.

**EXEMPLE 13**

Dans un autoclave en acier inoxydable de 125 $cm^3$, on introduit 0,1 g de $[RhCl(cyclooctadiène-1,5)]_2$ (0,406 milli-atome-gramme de rhodium), 1,23 g de TPPTS Na (2 milli-atome-gramme de $P^{3+}$) et 0,251 g de carbonate de sodium (2,36 m.moles). On purge trois fois à l'argon et on ajoute 20 g d'eau distillée, 7,83 g d'orthocrésol fraîchement distillé (72,4 m.moles) et 5,44 g d'isoprène (80 m.moles). On agite à 100°C pendant 16 heures.

Après refroidissement, décantation et concentration à 30°C sous pression réduite (15 mm de mercure; 2 kPa), on recueille 12,71 g de liquide jaune fluide.

Le taux de transformation de l'isoprène est de 85,8 % et celui de l'orthocrésol de 92,9 %.

L'analyse chromatographique montre la présence de 100 % de produits de monoaddition [méthyl-2 (méthyl-3 butène-2 yl)-4 phénol (10 %), méthyl-2 (méthyl-3 butène-3 yl)-4 phénol (70 %) et 20 % du mélange de méthyl-2 (méthyl-3 butène-2 yl)-6 phénol-méthyl-2 (méthyl-3 butène-3 yl)-6 phénol].

La structure des produits obtenus est confirmée par le spectre de résonance magnétique nucléaire du proton.

**EXEMPLE 14**

Dans un autoclave en acier inoxydable de 125 $cm^3$, on introduit 0,1 g de $[RhCl(cyclooctadiène-1,5)]_2$ (0,406 milli-atome-gramme de rhodium), 1,23 g de TPPTS Na (2 milli-atome-gramme de $P^{3+}$), 0,25 g de carbonate de sodium (2,36 m.moles) et 10,8 g de naphtol-2 (75 m.moles). On purge trois fois à l'argon et on ajoute 20 $cm^3$ d'un mélange 75/25 en volume d'eau et de méthanol et 10,9 de myrcène (80 m.moles). On agite à 100°C pendant 24 heures.

Après refroidissement, décantation et concentration à 30°C sous pression réduite (15 mm de mercure; 2 kPa), on récupère 11 g d'une huile jaune.

Le taux de transformation du myrcène est de 40 % et celui du naphtol-2 de 65 %.

L'analyse chromatographique montre la présence de [(diméthyl-3,7 octadiène-2,6 yl)-1 naphtol-2 (33 %) et (méthyl-7 méthylène-3 octène-6 yl)-1 naphtol-2 (67%)].

La structure des produits obtenus est confirmée par le spectre de résonance magnétique nucléaire du proton et par le spectre de masse.

**Revendications**

1 - Procédé de préparation de phénols substitués de formule générale:

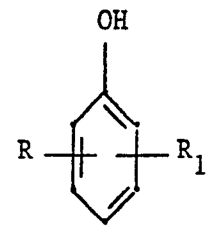

dans laquelle:
$R_1$ représente un radical

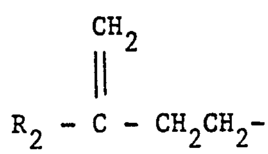 ou 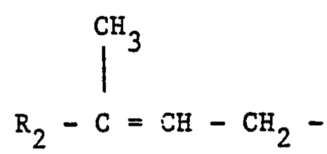

dans lequel $R_2$ représente un radical choisi parmi les radicaux acycliques ayant 1 à 20 atomes de carbone éventuellement substitués par un ou plusieurs radicaux hydroxy, alcoyloxycarbonyles, cyano ou formyle, et les radicaux phényle ou naphtyle éventuellement substitués par un ou plusieurs radicaux alcoyles contenant 1 à 20 atomes de carbone, et

R représente un atome d'hydrogène ou 1 à 4 substituants, identiques ou différents, choisis parmi les atomes d'halogène, les radicaux hydroxy éventuellement sous forme d'éthers ou d'esters, alcoyles, nitro, formyle, éventuellement sous forme d'acétal, acétyle, benzoyle, amino, alcoylamino, dialcoylamino ou alcoyloxycarbonyles, étant entendu que deux des symboles R peuvent former avec le noyau phényle un cycle aromatique condensé et étant entendu que le substituant $R_1$ est en position ortho ou para de la fonction phénol, par action d'un butadiène de formule générale:

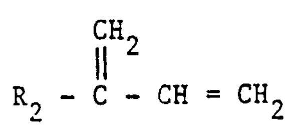

dans laquelle $R_2$ est défini comme précédemment, sur un phénol de formule générale:

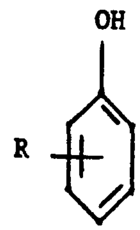

dans laquelle R est défini comme précédemment, étant entendu qu'au moins une position en ortho ou en para du radical hydroxy n'est pas substituée, caractérisé en ce que l'on opère dans l'eau en présence d'un catalyseur à base de rhodium soluble dans l'eau ou capable de passer en solution dans l'eau par réaction de coordination avec une phosphine soluble dans l'eau, de telle manière que le nombre d'atomes-gramme de rhodium par litre de solution réactionnelle est compris entre $10^{-4}$ et 1 et que le nombre d'atomes-gramme de phosphore trivalent rapporté à un atome-gramme de rhodium est compris entre 0,1 et 200, et éventuellement en présence d'une base minérale ou organique.

2 - Procédé selon la revendication 1 caractérisé en ce que les radicaux acycliques définis par $R_2$ sont choisis parmi les radicaux $CnH_{2+1}$; $CnH_{2n-1}$; $CnH_{2n-3}$ et $CnH_{n-1}$ où n est un nombre entier compris entre 1 et 20 inclusivement.

3 - Procédé selon l'une des revendications 1 ou 2 caractérisé en ce que le rhodium est mis en oeuvre sous forme d'un composé choisi parmi les sels inorganiques ou organiques et les complexes du rhodium choisis parmi $RhCl_3$, $RhBr_3$, $Rh_2O$, $Rh_2O_3$, $Rh(NO_3)_3$, $Rh_2(CH_3COO)_4$, $Rh(CH_3COCHCOCH_3)_3$, $[RhCl(cyclooctadiène\text{-}1,5)]_2$, $[RhCl(CO)_2]_2$ et $RhCl_3(C_2H_5NH_2)_3$.

4 - Procédé selon la revendication 3 caractérisé en ce que le composé du rhodium est choisi parmi $RhCl_3$ et $[RhCl(cyclooctadiène\text{-}1,5)]_2$.

5 - Procédé selon l'une des revendications 1 ou 2 caractérisé en ce que la phosphine soluble dans l'eau est choisie parmi les phosphines de formule générale:

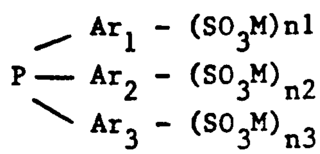

dans laquelle $Ar_1$, $Ar_2$ et $Ar_3$, identiques ou différents, représentent chacun un radical phénylène ou naphtylène éventuellement substitué, M est un reste cationique minéral ou organique choisi parmi les cations sodium, potassium, calcium, baryum, ammonium et ammonium quaternaire, $n_1$, $n_2$ et $n_3$, identiques ou différents, sont des nombres entiers supérieurs ou égaux à 0 et inférieurs ou égaux à 3, l'un au moins étant supérieur à 1.

6 - Procédé selon la revendicatiom 5 caractérisé en ce que la phosphine soluble dans l'eau est le sel de sodium de la tri(m-sulfophényl)phosphine.

7 - Procédé selon l'une des revendications 1, 2, 3, 4, 5 ou 6, caractérisé en ce que l'on utilise une quantité d'eau suffisante pour dissoudre le totalité du catalyseur et au moins une partie du phénol et du butadiène substitué mis en oeuvre.

8 - Procédé selon la revendication 1 caractérisé en ce que l'on opère en présence d'un co-solvant choisi parmi les alcools aliphatiques contenant 1 à 4 atomes de carbone.

9 - Procédé selon la revendication 8 caractérisé en ce que l'on utilise une quantité d'alcool aliphatique qui remplace au maximum la moitié de la quantité d'eau nécessaire à la mise en oeuvre du procédé sans co-solvant.

10 - Procédé selon la revendication 1 caractérisé en ce que l'on opère en présence d'une base minérale ou organique choisie parmi les hydroxydes, carbonates et bicarbonates de métaux alcalins ou alcalino-terreux et

les amines tertiaires aliphatiques ou aromatiques.

11 - Procédé selon l'une des revendications 1, 2, 3, 4, 5, 6, 7, 8, 9 ou 10, caractérisé en ce que l'on opere à une temperature inférieure à 200° C.

**Patentansprüche**

1. Verfahren zur Herstellung von substituierten Phenolen der allgemeinen Formel:

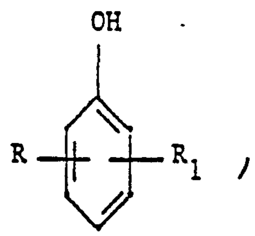

in welcher:

$R_1$ einen Rest

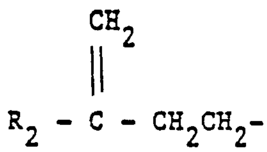 oder 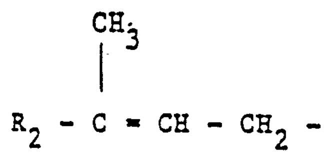

bedeutet, in welchem $R_2$ einen Rest bedeutet, der ausgewählt ist unter den acyclischen Resten mit 1 bis 20 Kohlenstoffatomen, gegebenenfalls substituiert durch einen oder mehrere Hydroxy-Alkyloxycarbonyl-, Cyano- oder Formylreste, und den gegebenenfalls durch ein oder mehrere Alkylreste mit 1 bis 20 Kohlenstoffatomen substituierten Formyl- oder Naphthylresten, und

R ein Wasserstoffatom oder 1 bis 4 Substituenten, die gleich oder verschieden sind, bedeutet, die ausgewählt sind unter den Halogenatomen, den Hydroxyresten, gegebenenfalls in Form von Äthern oder Estern, Alkyl-, Nitro-, Formylresten, gegebenenfalls in Form von Acetal, Acetyl-, Benzoyl-, Amino-, Alkylamino-, Dialkylamino- oder Alkyloxycarbonylresten, wobei selbstverständlich zwei der Symbole R mit dem Phenylkern einen kondensierten aromatischen Ring bilden können und der Substituent $R_1$ in ortho- oder para-Stellung der Phenolfunktion ist, durch Umsetzung eines Butadiens der allgemeinen Formel:

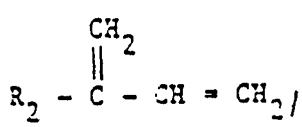

in welcher $R_2$ wie oben definiert ist, mit einem Phenol der allgemeinen Formel

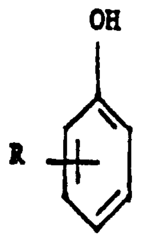

,

in welcher R wie oben definiert ist, wobei selbstverständlich mindestens eine ortho- oder para-Stellung des Hydroxyrestes nicht substituiert ist, dadurch gekennzeichnet, daß man in Wasser in Gegenwart eines Katalysators auf Basis von Rhodium, der in Wasser lösbar ist oder in Wasser durch Koordinationsreaktion mit einem in Wasser lösbaren Phosphin in Lösung gehen kann, sodaß die Anzahl von Grammatomen Rhodium pro Liter Reaktionslösung zwischen $10^{-4}$ und 1 ist und daß die Zahl von Grammatomen von dreiwertigem Phosphor, bezogen auf ein Grammatom Rhodium, zwischen 0,1 und 200 ist, und gegebenenfalls in Gegenwart einer anorganischen oder organischen Base arbeitet.

2. Verfahren nach Anspruch 1, dadurch gekennzeichnet, daß die durch $R_2$ definierten acyclischen Reste ausgewählt sind unter den Resten $C_nH_{2n+1}$; $C_nH_{2n-1}$; $C_nH_{2n-3}$ und $C_nH_{n-1}$, worin n eine ganze Zahl zwischen 1 und einschließlich 20 ist.

3. Verfahren nach einem der Ansprüche 1 oder 2, dadurch gekennzeichnet, daß das Rhodium in Form einer Verbindung eingesetzt wird, die ausgewählt ist unter den anorganischen oder organischen Salzen und den Komplexen von Rhodium, ausgewählt unter $RhCl_3$, $RhBr_3$, $Rh_2O$, $Rh_2O_3$, $Rh(NO_3)_3$, $Rh_2(CH_3COO)_4$, $Rh(CH_3COCHCOCH_3)_3$, $[RhCl(1,5\text{-cyclooctadien})]_2$, $[RhCl(CO)_2]_2$ und $RhCl_3(C_2H_5NH_2)_3$.

4. Verfahren nach Anspruch 3, dadurch gekennzeichnet, daß die Rhodiumverbindung ausgewählt ist unter $RhCl_3$ und $[RhCl(1,5\text{-cyclooctadien})]_2$.

5. Verfahren nach einem der Ansprüche 1 oder 2, dadurch gekennzeichnet, daß das in Wasser lösliche Phosphin ausgewählt wird unter den Phosphinen der allgemeinen Formel:

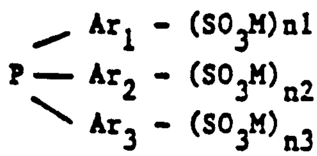

,

in welcher $Ar_1$,$Ar_2$ und $Ar_3$, gleich oder verschieden sind, jeweils einen gegebenenfalls substituierten Phenylen- oder Naphthylenrest bedeuten, M ein anorganischer oder organischer kationischer Rest ist, ausgewählt unter den Kationen Natrium, Kalium, Kalzium, Barium, Ammonium und quaternäres Ammonium, $n_1$, $n_2$ und $n_3$, die gleich oder verschieden sind, ganze Zahlen größer oder gleich 0 und kleiner oder gleich 3 sind, wobei mindestens eine größer 1 ist.

6. Verfahren nach Anspruch 5, dadurch gekennzeichnet, daß das in Wasser lösliche Phosphin das Natriumsalz des Tri(m-sulfophenyl)phosphins ist.

7. Verfahren nach einem der Ansprüche 1, 2, 3, 4, 5 oder 6, dadurch gekennzeichnet, daß man eine Wassermenge verwendet, die ausreicht, um den Katalysator insgesamt und mindestens einen Teil des eingesetzten substituierten Phenols und Butadiens zu lösen.

8. Verfahren nach Anspruch 1, dadurch gekennzeichnet, daß Man in Gegenwart eines Co-Lösungsmittels, ausgewählt unter den aliphatischen Alkoholen mit 1 bis 4 Kohlenstoffatomen, arbeitet.

9. Verfahren nach Anspruch 8, dadurch gekennzeichnet, daß man eine Menge aliphatischen Alkohols verwendet, die maximal die Hälfte der zur Durchführung des Verfahrens ohne Co-Lösungsmittel erforderlichen Wassermenge ersetzt.

10. Verfahren nach Anspruch 1, dadurch gekennzeichnet, daß man in Gegenwart einer anorganischen oder organischen Base, ausgewählt unter den Hydroxiden, Carbonaten und Bicarbonaten von Alkali- oder

Erdalkalimetallen und den aliphatischen oder aromatischen tertiären Aminen, arbeitet.

11. Verfahren nach einem der Ansprüche 1, 2, 3, 4, 5, 6, 7, 8, 9 oder 10, dadurch gekennzeichnet, daß man bei einer Temperatur unter 200° C arbeitet.

**Claims**

1. Process for the preparation of substituted phenols of general formula:

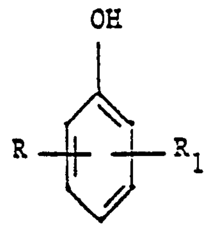

in which

$R_1$ denotes a radical

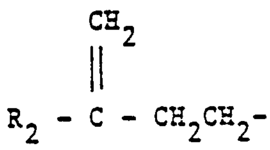

or

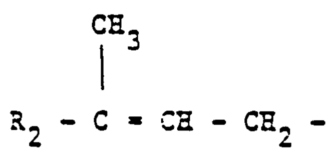

in which $R_2$ denotes a radical chosen from acyclic radicals containing 1 to 20 carbon atoms optionally substituted by one or more hydroxy, alkoxycarbonyl, cyano or formyl radicals, and phenyl or naphthyl radicals optionally substituted by one or more alkyl radicals containing 1 to 20 carbon atoms, and

R denotes a hydrogen atom or 1 to 4 substituents, identical or different, chosen from halogen atoms, hydroxy radicals optionally in the form of ethers or esters, alkyl, nitro, formyl, optionally in acetal form, acetyl, benzoyl, amino, alkylamino, dialkylamino or alkoxycarbonyl radicals, it being understood that two of the symbols R may form a condensed aromatic ring with the phenyl nucleus and it being understood that the substituent $R_1$ is in the ortho or para position with respect to the phenol group, by the action of a butadiene of general formula:

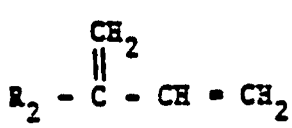

in which $R_2$ is defined as above on a phenol of general formula:

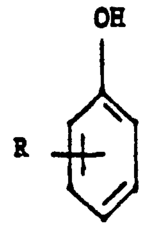

in which R is defined as above it being understood that at least one position ortho or para to the hydroxy radical is unsubstituted, characterized in that the operation is carried out in water in the presence of a rhodium-based catalyst which is soluble in water or capable of dissolving in water by means of a coordination reaction with a water-soluble phosphine, so that the number of gram-atoms of rhodium per litre of reaction solution is between $10^{-4}$ and 1 and that the number of gram-atoms of trivalent phosphorus added to one gram-atom of rhodium is between 0.1 and 200, and optionally in the presence of an inorganic or organic base.

2. Process according to Claim 1, characterized in that the acyclic radicals defined by $R_2$ are chosen from the radicals $CnH_{2n+1}$; $CnH_{2n-1}$; $CnH_{2n-3}$ and $CnH_{n-1}$, where n is an integer from 1 to 20 inclusively.

3. Process according to either of Claims 1 and 2, characterized in that the rhodium is used in the form of a compound chosen from inorganic or organic rhodium salts and complexes chosen from $RhCl_3$, $RhBr_3$, $Rh_2O$, $Rh_2O_3$, $Rh(NO_3)_3$, $Rh_2(CH_3COO)_4$, $Rh(CH_3COCHCOCH_3)_3$, $[RhCl(1,5\text{-cyclooctadiene})]_2$, $[RhCl(CO)_2]_2$ and $RhCl_3(C_2H_5NH_2)_3$.

4. Process according to Claim 3, characterized in that the rhodium compound is chosen from $RhCl_3$ and $[RhCl(1,5\text{-cyclooctadiene})]_2$.

5. Process according to either of Claims 1 and 2, characterized in that the water-soluble phosphine is chosen from the phosphines of general formula:

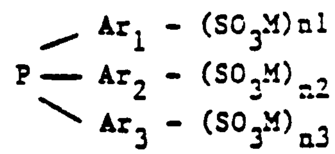

in which each of $Ar_1$, $Ar_2$ and $Ar_3$, which are identical or different, denotes an optionally substituted phenylene or naphthylene radical, M is an inorganic or organic cationic residue chosen from the sodium, potassium, calcium, barium, ammonium and quaternary ammonium cations, and $n_1$, $n_2$ and $n_3$, which are identical or different, are integers greater than or equal to 0 and smaller than or equal to 3, at least one being greater than 1.

6. Process according to Claim 5, characterized in that the water-soluble phosphine is the sodium salt of tri(m-sulphophenyl)phosphine.

7. Process according to one of Claims 1, 2, 3, 4, 5 and 6, characterized in that a sufficient quantity of water is used to dissolve all the catalyst and at least some of the phenol and of the substituted butadiene employed.

8. Process according to Claim 1, characterized in that the operation is carried out in the presence of a cosolvent chosen from aliphatic alconols containing 1 to 4 carbon atoms.

9. Process according to Claim 8, characterized in that a quantity of aliphatic alcohol is used which replaces at most one half of the quantity of water requred for performing the process without a cosolvent.

10. Process according to Claim 1, characterized in that the operation is carried out in the presence of an inorganic or organic base chosen from alkali metal or alkaline-earth metal hydroxides, carbonates and bicarbonates and aliphatic or aromatic tertiary amines.

11. Process according to one of Claims 1, 2, 3, 4, 5, 6, 7, 8, 9 and 10, characterized in that the operation is carried out at a temperature below 200° C.